# EUROPEAN PATENT APPLICATION

(11) **EP 3 520 787 A1**
(43) Date of publication of application: **07.08.2019**
(21) Application number: 18154584.9
(22) Date of filing: 01.02.2018
(51) Int. Cl.: A61K 31/353, A61K 31/7048, A61P 17/18, A61Q 17/04

(54) **COMPOSITION AND USE THEREOF AGAINST DRUGS INDUCED PHOTOTOXICITY**

(71) Applicant: AC BioScience SA, 1024 Ecublens (CH)
(72) Inventor: AUCLAIR, Christian, 78730 Saint-Arnoult en Yvelines (FR)
(74) Representative: KATZAROV S.A.

(57) **Abstract**

The invention provides the pharmaceutical composition and use thereof for treating or lessening the symptoms of, or preventing the symptoms of drug-induced phototoxicity or photosensitivity.

## Description

### FIELD OF THE INVENTION

The invention provides the pharmaceutical composition and use thereof for treating or lessening the symptoms of, or preventing the symptoms of drug-induced phototoxicity or photosensitivity.

### BACKGROUND OF THE INVENTION

Drug-induced phototoxicity or photosensitivity or photosensitising medications can cause unexpected sunburn or a dry, bumpy or blistering rash on sun-exposed skin (face, neck, arms, backs of hands and often lower legs and feet). Drug- and chemical-induced photosensitivity occurs when a drug or chemical agent combines with UV radiation to cause a phototoxic or less frequently photoallergic reaction. These agents are called photosensitisers and can be topical agents or medications that are taken orally (Drucker et al. 2011).

The clinical features of drug-induced photosensitivity vary according to the photosensitising agent involved and the type of reaction it causes in the skin. Ultraviolet (UVA) or in some cases, visible light activates the photosensitizing drug to emit energy that may damage adjacent skin tissue resulting in an intensified sunburn with skin peeling. Factors influencing the intensity and incidence of drug-induced phototoxicity include: 1) the concentration, absorption, and pharmacokinetics of the drug. Higher doses of lipophilic drugs known to cause this reaction have a higher incidence, and 2) the "dose" of sunlight (i.e., quantity and spectrum of sunlight).

Phototoxicity is characterized by a rapid onset of erythema, pain, prickling, or burning sensation of areas exposed to the sun, with peak symptoms occurring 12-24 hours after the initial exposure. The hallmark of this reaction is the appearance of a sunburn-like reaction on areas of skin with the greatest exposure to sunlight. These reactions do not initially involve the immune system; therefore, prior exposure or sensitization to a drug is not necessary for this reaction to occur. Phototoxic reactions typically involve the following:
▪ Skin reaction occurs minutes to hours after exposure to agent and light
▪ Appears as an exaggerated sunburn reaction (reddening and swelling)
▪ Vesicles, blisters and bullae may occur in severe reactions (pseudoporphyria)
▪ May or may not be itchy
▪ Less commonly, skin may change colour, e.g. blue-green pigmentation is associated with amiodarone
▪ Reaction is limited to sun-exposed skin
▪ Photo-onycholysis (separation of the distal nail plate from the nail bed) may arise with many oral photosensitising medications and may be the only sign of phototoxicity in dark-skinned individuals

Because of the occurrence of drug-induced photosensitization process, the skin, exposed to sunlight is submitted to a high flux of both oxy-radicals and singlet oxygen. Despite the presence of various natural endogenous protective systems, this oxidative stress rapidly results in the alteration of the different skin components.

In contrast to singlet oxygen, it should be noticed that superoxide anion is not a powerful oxidant except under its protonated form (HO₂°) but this entity is negligible at physiological pH, since the pKa of O₂°- is close to 4.8. However, superoxide anion readily dismutates into hydrogen peroxide which can subsequently produce the hydroxyl radical (OH°) through a one-electron reduction as follows:

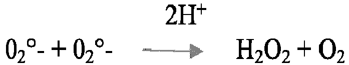

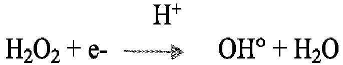

With singlet oxygen, hydroxyl radical is one of the most powerful oxidizing agents present in biological tissues.

In this context, many strategies have been proposed to slow the skin inflammation resulting from phototoxicity, most of them consisting in topic formulation containing oxy-radical scavengers. However, because of the involvement of both the Type-I and Type-II mechanisms in the photosensitizer's activation under sunlight exposure, oxy-radicals scavenger alone could not protect efficiently the skin and the presence of a singlet oxygen quencher is as well required.

Therefore, there is still a need for a composition that can efficiently protect the skin against drug-induced phototoxicity.

### SUMMARY OF THE INVENTION

An aspect of the present invention provides a pharmaceutical composition comprising therapeutically effective amounts of
- dihydroflavone or dihydroflavonol,
- (+)-catechin or a derivative thereof,
- terpene alcohol, and
- pharmaceutically acceptable excipients and/or carriers.

Another aspect of the present invention provides the pharmaceutical composition of any one of claims 1-5 for use in a method for treating or lessening the symptoms of, or preventing the symptoms of drug-induced phototoxicity or photosensitivity.

### BRIEF DESCRIPTION OF THE FIGURES

**Figure 1** shows Oxygeno-dependant mechanisms of drugs (chemicals)-induced photoxicity. Type I photosensitization results in the production of oxy-radical intermediates whereas type II involving energy transfer results in the production of the singlet oxygen.
**Figure 2** shows the basic structure of flavonoids.

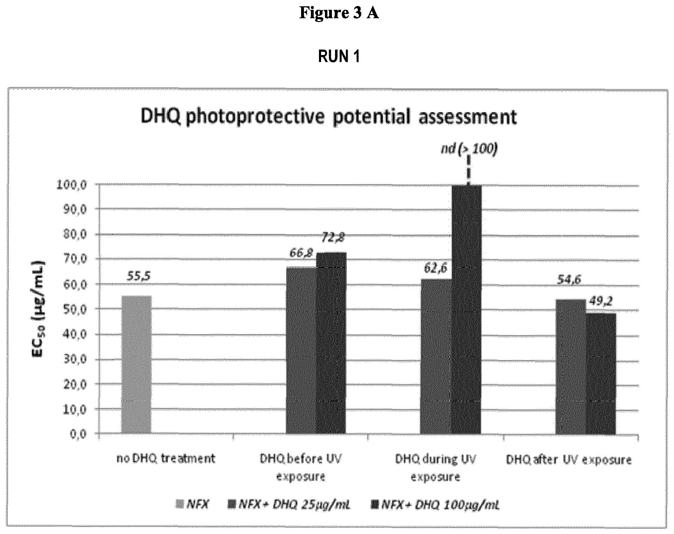

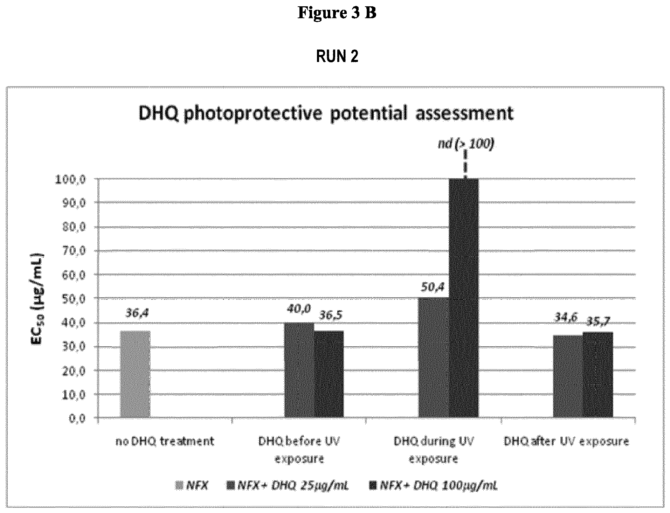

**Figure 3** **A & B** show results of two runs show that DHQ treatment at 100µg/ml during UVA at 5 J/cm2 exposure increases NFX EC50 =>DHQ (BN0901-2) seems to protect against UVA cellular damages when treatment is performed during UV exposure in the presence of norfloxacin.
**Figure 4** shows experiments were performed as described for NFX. In the control experiment (no treatment) the IC50 of CPZ was 0.33 µg/ml. Increasing this value indicates of protective effect on cells.
**Figure 5** shows dihydroquercetin mediated inhibition of polymorphonuclear neutrophils oxidative burst

### DETAILED DESCRIPTION OF THE INVENTION

All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of" and/or "consisting essentially of".

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

As used herein the terms "subject" and "patient" are well-recognized in the art, and, are used herein to refer to a mammal, and most preferably a human. In some embodiments, the subject is a subject in need of treatment or a subject with drug-induced phototoxicity or photosensitivity disorder. The term does not denote a particular age or sex. Thus, adult and newborn subjects, whether male or female, are intended to be covered.

As used herein the term "pharmaceutically acceptable excipients and/or carriers" means that the compositions or components thereof so described are suitable for use in contact with skin of human, or suitable for any other means of administration to human body without undue toxicity, incompatibility, instability, irritability, allergic response, and the like.

As used herein the term "topical" or "topically" refers to the application of the composition of the present invention onto the surface of the skin and/or a portion thereof.

As used herein the term "administration" or "administering" to human body refers to any means of introducing the composition of the present invention onto and/or into the human body or a portion thereof (such as topical administration into and/or onto the skin or portion of the skin or topical application on the skin or portion thereof).

The term "therapeutically effective amount," as used herein, refers to any amount of a specific component or combination of components that will cause a reduction of symptoms, disappearance of the symptoms or relief from symptoms related to drug-induced phototoxicity, when applied, either once, or repeatedly over time. Therapeutically effective amounts can be readily determined by persons skilled in the art using routine experimentation and using tests and measures commonly employed in the art, or can be based upon the subjective response of patients undergoing treatment.

There is a huge number of drugs that have been reported to cause photoxic reactions upon light exposure with various incidences. Drugs that induce phototoxicity or photosensitivity are for example:
**Fluoroquinolones:**
   ciprofloxacin (*Cipro*), gemifloxacin (*Factive*), levofloxacin (*Levaquin*), lomefloxacin (*Maxaquin*), moxifloxacin (*Avelox*), norfloxacin (*Noroxin*), ofloxacin (*Floxin*)
**Antipsychotics:**
   Antipsychotics,Phenothiazines: chlorpromazine (*Thorazine*), fluphenazine (*Prolixin*), perphenazine (*Trilafon*), prochlorperazine (*Compazine*), thioridazine (*Mellaril*), trifluoperazine (*Stelazine*) Antipsychotics, Other: clozapine (*Clozaril*), haloperidol (*Haldol*), loxapine (*Loxitane*), olanzapine (*Zyprexa*), quetiapine (*Seroquel*), risperidone (*Risperdal*), thiothixene (*Navane*), ziprasidone (*Geodon*)
**Antidepressants:**
   Tricyclic Antidepressants: amitriptyline (*Elavil*), amoxapine (*Asendin*), clomipramine (*Anafranil*), desipramine (*Norpramin*), doxepin (*Sinequan*), imipramine (*Tofranil*), maprotiline (*Ludiomil*), nortriptyline (*Pamelor*), protriptyline (*Vivactil*), trimipramine (*Surmontil*) Selective serotonin reuptake inhibitors:
      citalopram (*Celexa*), escitalopram (*Lexapro*), fluoxetine (*Prozac, Sarafem*), fluvoxamine (*Luvox*), paroxetine (*Paxil*), sertraline (*Zoloft*) Antidepressant, Other: bupropion (*Wellbutrin*), mirtazapine (*Remeron*), nefazodone (*Serzone*), trazodone (*Desyrel*), venlafaxine (*Effexor*)
**Antihistamines:**
   Cetirizine (*Zyrtec*), cyproheptadine (*Periactin*),diphenhydramine (*Benadryl*), loratadine (*Claritin*), promethazine (*Phenergan*)
**Antitumors:**
   Bexarotene (*Targretin*), capecitabine (*Xeloda*), dacarbazine (*DTIC*), epirubicin (*Ellence*), fluorouracil (*5-FU*), interferon alfa (*Intron A, Alferon-N*), methotrexate (*Mexate*), pentostatin (*Nipent*), procarbazine (*Matulane*), tretinoin, oral (*Vesanoid*), vinblastine (*Velban, Velbe*)
**Cardiovasculars** :
   Thiazide diuretics:
      Bendroflumethiazide (*Corzide*), chlorthalidone (*Thalitone*), hydrochlorothiazide (*Microzide*), hydroflumethiazide (*Diucardin*), indapamide (*Lozol*), methyclothiazide (*Enduron*), metolazone (*Zaroxolyn*), polythiazide (*Renese*)
   Diuretics, Other:
      furosemide (*Lasix*), triamterene (*Dyrenium*)
**Antihypertensives:**
   captopril (*Capoten*), diltiazem (*Cardizem, Tiazac*), enalapril (*Vasotec*), nifedipine (*Procardia*), sotalol (*Betapace*) Statins: fluvastatin (*Lescol*), lovastatin (*Mevacor*), pravastatin (*Pravachol*), simvastatin (*Zocor*)
**Other drugs:**
   amiodarone (*Cordarone, Pacerone*), fenofibrate (*Tricor*), quinidine

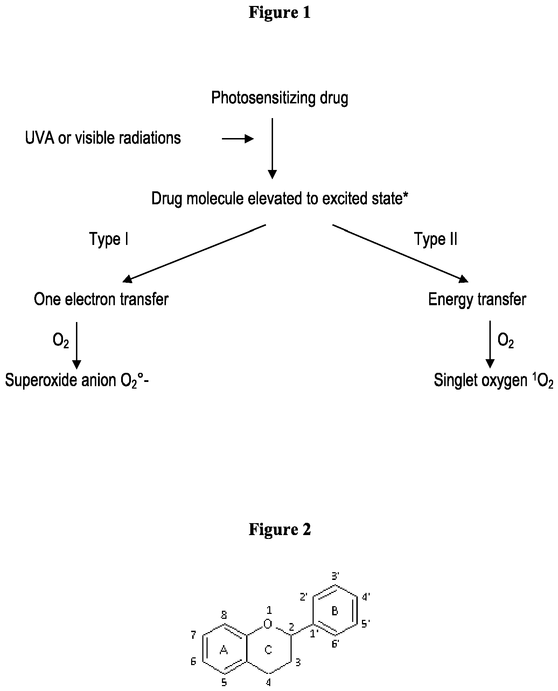

The molecular pathogenic mechanisms of drug-induced phototoxicity is summarized on Figure 1. It was clearly shown that reactive oxygen species, including oxy-radicals and singlet oxygen (see Figure 1), might play an important role in drug-induced phototoxicity. This alternative process (superoxide anion versus singlet oxygen) as depicted in Figure 1 is under the control of the environment of the reaction, a high oxygen concentration favoring the singlet oxygen production. In fact, both oxy-radicals and singlet oxygen (¹O₂) are generated by a number of enzymes as well as by UVA (320 - 400 nm) or visible light in the presence of a sensitizer and have been proposed as damaging agents in a number of pathologies including cataract, sunburn, and skin cancers. Lipids, proteins, amino acids Cys, Met, Trp, Tyr and His side chains in particular, are major targets for oxy-radicals and ¹O₂ as a result of their abundance and high rate constants for reaction. It should be noticed as well that singlet oxygen appears to be a major species produced in cells by UVA which results in oxidations in the cells and skin aging.

An aspect of the present invention provides a pharmaceutical composition that is used for skin protection against drug-induced phototoxicity or drug-induced photosensitization. In some embodiments, protection relates to treating or lessening the symptoms of, or preventing the symptoms of drug-induced phototoxicity or photosensitivity.

According to an embodiment, the pharmaceutical composition of the invention comprises therapeutically effective amounts of
- dihydroflavone or dihydroflavonol,
- (+)-catechin or a derivative thereof,
- terpene alcohol, and
- pharmaceutically acceptable excipients and/or carriers.

In the context of the present invention, it is important that both dihydroflavones and dihydroflavonols as well as catechins have the C ring saturated, i.e. no double bond between positions 2 and 3 (see Figure 2). Thus these chemical compounds do not interact with singlet oxygen to generate a toxic reactive endoperoxide. The flavanones can be multi-hydroxylated, and several hydroxyl groups can be glycosylated and/or methylated. Some have unique patterns of substitution, for example, furanoflavanones, prenylated flavanones, pyranoflavanones or benzylated flavanones, giving a great number of substituted derivatives. Dihydroflavonols, are the 3-hydroxy derivatives of flavanones; they are an highly diversified and multisubstituted subgroup. Catechins and derivatives thereof have two benzene rings (called the A- and B-rings) and a dihydropyran heterocycle (the C-ring) with a hydroxyl group on carbon 3. The A ring is similar to a resorcinol moiety while the B ring is similar to a catechol moiety. There are two chiral centres on the molecule on carbons 2 and 3 (see Figure 2). Therefore, it has four diastereoisomers. Two of the isomers are in trans configuration and are called *catechin* and the other two are in cis configuration and are called *epicatechin.* Preferably catechin is (+)-catechin and a derivative thereof. Derivatives of (+)-catechin are for example (+)-catéchine C, (+)-gallocatéchine GC.

In a preferred embodiment of the present invention, dihydroflavone or dihydroflavonol is selected from the group comprising Dihydroquercetin (DHQ), Dihydrokaempferol, Butin, Eriodictyol, Hesperetin, Hesperidin, Homoeriodictyol, Isosakuranetin, Naringenin, Naringin, Pinocembrin, Poncirin, Sakuranetin, Sakuranin, Sterubin. The most preferably dihydroflavonol is Dihydroquercetin (DHQ).

In a preferred embodiment of the present invention, the terpene alcohol is selected from the group comprising a monoterpene alcohol, a sesquiterpene alcohol or a diterpene alcohol and combinations of one or more thereof. Preferably, the one or more terpene alcohol is selected from the group comprising cedrenol, cedrols, geraniol, nerolidol, bisabolols, citronellol, nerol, terpineol, linalool, menthol, pulegol, carveol, pinocampheol, myrcenol, isopulegol, farnesol, lanceol, santalols, vetiverol, viridiflorol, valerianol, tumerols, patchoulol, occidol, nootkatol, jinkoh eremol, hanamyol, guaicol germacradienol, fokienol, eudesmols, and cadinols, an active optical or steric isomer of these compounds and combinations of one or more thereof. The most preferably, the terpene alcohol is bisabolol.

According to a specific embodiment of the present invention, the pharmaceutical composition comprises therapeutically effective amounts of
- Dihydroquercetin,
- (+)-catechin,
- Bisabolol, and
- pharmaceutically acceptable excipients and/or carriers.

The presence of above-mentioned active ingredients in the pharmaceutical composition of the invention provides unique properties to said composition and enables efficient protection of the skin against drug-induced phototoxicity or drug-induced photosensitization because the pharmaceutical composition of the invention is active against both Type-I and Type-II mechanisms in the photosensitizer's activation under sunlight exposure. Indeed, dihydroflavones or dihydroflavonols (such as Dihydroquercetin) act both as oxy-radical and singlet oxygen scavenger without the formation of oxidizing endoperoxides; (+)-Catechins and derivatives thereof have similar activity to Dihydroquercetin but in addition modulate enzyme function and genes expression resulting in decrease in oxy-radical's generation and increase in antioxidant protection; and the terpene alcohol (such as bisabolol) display global anti-inflammatory properties, inhibits the neutrophils degranulation and facilitates the diffusion of active molecules to the dermal area. Therefore the pharmaceutical composition of the invention limits the reactive oxygen species causing skin damages and limits the associated skin inflammation. The physicochemical and biologicals properties of the three active ingredients are summarized in Table 1.

**Table 1. Physicochemical and biologicals properties of the active ingredients present in the pharmaceutical composition of the invention. DHQ = dihydroquercetin, SOD = superoxide dismutase.**

| **Molecules** | Oxy-radicals scavenger | Singlet oxygen quencher | ↓ | Neutrophils activation | SOD induction | ↑ | Drug diffusion |
|---|---|---|---|---|---|---|---|
| **DHQ** | + | + | + | | - | - | |
| **(+) catechin** | + | + | + | | + | - | |
| **Bisabolol** | - | - | + | | - | + | |

According to another embodiment, the invention provides the composition comprising
- dihydroflavone or dihydroflavonol,
- (+)-catechin or a derivative thereof,
- terpene alcohol, and
- excipients and/or carriers.
The composition of the invention can be used for cosmetic applications.

The pharmaceutical compositions of the invention comprise also pharmaceutically acceptable excipients and/or carriers for topical use, as are used conventionally in such compositions, for example preservatives, antioxidants, bactericides, fungicides, solvents, perfumes, substances for preventing foaming, dyestuffs, pigments which have a colouring effect, thickeners, propellants, surfactant substances, emulsifiers, softening, moisturizing and/or moisture-retaining substances, distilled water, fats, oils, waxes or other conventional constituents of a topical composition, such as alcohols, polyols, polymers, foam stabilizers, electrolytes, organic solvents or silicone derivatives. The necessary amounts of the pharmaceutically acceptable excipients and/or carriers can, based on the desired product, easily be chosen by a person skilled in the art.

According to an embodiment of the present invention, the pharmaceutical composition of the invention is a cream, water based O/W, comprising the following excipients and/or carriers: water, sweet almond oil, caprylic/capric triglyceride, olus oil, butyrospermum parkII butter, dicaprylyl ether, glycerin, propanediol, alcohol denat, sorbitol, cetearyl alcohol, glyceryl stearate, glyceryl stearate citrate, polyglyceryl-3 methylglucose distearate, xanthan gum, sodium dehydroacetate, sodium benzoate, phenoxyethanol, citric acid.

The pharmaceutical compositions of the invention can take various forms, depending on topical application. For example, the pharmaceutical compositions for topical administration can be in the form of ointments, lotions, creams, foams, gels, solutions patches or sprays. The pharmaceutical compositions can also be incorporated into dedicated applicators, such as saturated pads, to facilitate administration to the skin.

The pharmaceutical compositions of the invention can be packaged to provide a single dose or multiple doses, and to provide a convenient means of transport, handling, and administration. The pharmaceutical compositions can be also packaged in such a way as to protect the composition from oxidation, bacterial contamination, or other forms of deterioration or degradation. For example, the pharmaceutical compositions of the invention for topical administration can be packaged into crimped tubes, airless containers, or sealed foil-lined packets, which may optimally contain enough of the composition for a single application, or a limited number of applications. The pharmaceutical compositions of the invention for topical administration can be packaged in larger containers designed for multiple applications. When packaged in such larger containers, those containers may be equipped with pumps or other mechanisms designed to facilitate the delivery of an appropriate volume of the composition, while reducing the likelihood of contamination or oxidation.

The pharmaceutical compositions of the invention are intended for use on mammalian skin, including, for example, the skin of humans, domestic pets, livestock and other farm animals. When used on human subjects, or human patients in need of such treatment, the human patients may be of any age or gender, although specific compositions may be developed for treating human patients within specific age ranges, or of a particular gender.

The pharmaceutical compositions of the invention are intended to treat skin lesions (disorders or conditions of the skin), specifically the symptoms, resulting from drug-induced phototoxicity or photosensitivity, such as erythema, pain, prickling, rash, burning sensation of areas of skin exposed to the sunlight, and/or the appearance of a sunburn-like reaction on areas of skin exposed to the sunlight. Typically, but not limited to, sun-exposed areas of skin are face, neck, arms, backs of hands and often lower legs and feet. Rash can be typically dry, bumpy or blistering rash.

The pharmaceutical compositions of the invention can be also be used prophylactically, in order to prevent, protect and/or lessen the symptoms of drug-induced phototoxicity or photosensitivity.

Another aspect of the present invention provides the pharmaceutical composition of the invention for use in a method for treating or lessening the symptoms of, or preventing the symptoms of drug-induced phototoxicity or photosensitivity. In a specific embodiment, the present invention provides the pharmaceutical composition of the invention for use in a method for treating the symptoms of drug-induced phototoxicity or photosensitivity. In another specific embodiment, the present invention provides the pharmaceutical composition of the invention for use in a method for lessening the symptoms of drug-induced phototoxicity or photosensitivity. In a further specific embodiment, the present invention provides the pharmaceutical composition of the invention for use in a method for preventing the symptoms of drug-induced phototoxicity or photosensitivity.

The symptoms of drug-induced phototoxicity or photosensitivity are selected from erythema, pain, prickling, or burning sensation of areas exposed to the sun, and the appearance of a sunburn-like reaction on areas of skin with the greatest exposure to sunlight.

In some embodiments, the method comprises applying the pharmaceutical composition to the affected skin of a patient once a day. In other embodiments the method comprises applying the pharmaceutical composition to the affected skin of a patient multiple times a day.

Further aspect of the present invention provides a method of treating or lessening the symptoms of, or preventing the symptoms of drug-induced phototoxicity or photosensitivity, comprising administering the pharmaceutical composition of the invention to the affected skin of a patient.

In some embodiments, the method comprises applying the pharmaceutical composition to the affected skin of a patient once a day. In other embodiments the method comprises applying the pharmaceutical composition to the affected skin of a patient multiple times a day.

The methods of treatment to be employed with the pharmaceutical compositions of the invention will vary depending upon the disorder, or condition, or symptom to be treated, and its severity. The methods will also vary depending upon the nature of the subject to be treated; their species, gender, and age, etc. Optimal methods of treatment, including the choice of specific formulation, the form of that formulation, the frequency of administration, and the duration of treatment will be adjusted according to the response of the patient, and the efficacy of the treatment, as will be judged by the patient themselves, or by a health care provider who is directing the treatment. Specific details regarding the methods of treatment can be defined by a health care provider overseeing the treatment, or by the patient, as results are obtained. Effective results will, in most cases, be achieved by topical application of the pharmaceutical compositions of the invention in a thin layer directly over the affected area or areas, or in the area where one seeks to obtain a desired result.

Depending upon the skin lesion, disorder, condition, or symptom to be treated, and its severity, and whether the treatment is being done for therapeutic or prophylactic reasons, effective results may be obtained with application rates of from one application every week, to once every day, to multiple applications per day. The duration of the treatment regimen can be adjusted according to the patient's needs and according to the disorder's response to the treatment. Treatment can either be discontinued, or its frequency lessened, once symptoms diminish or disappear. Alternatively, it may be advantageous for treatments to continue for a fixed period beyond the diminution or disappearance of symptoms, and in other cases, it may be advantageous for treatment to continue indefinitely as a prophylactic treatment in patients who suffer from chronic drug-induced phototoxicity.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims, and all changes which come within the meaning and range of equivalency are intended to be embraced therein.

The foregoing description will be more fully understood with reference to the following Examples. Such Examples, are, however, exemplary of methods of practising the present invention and are not intended to limit the application and the scope of the invention.

### EXAMPLES

### Assessment of Dihydroquercetin (DHQ) photoprotective potential

Regarding the antioxidant properties of DHQ, a protective effect of this compound against cells damages resulting from photosensitization (phototoxicity) induced by drugs such as quinolone antibiotics has been texted.

Lomefloxacin, norfloxacin, ofloxacin, and enoxacin (broad-spectrum antibiotics of fluoroquinolone group) are used for the treatment of Gram-positive and Gram-negative bacterial infections. Phototoxicity and possible mechanism of their action was assessed under the exposure of ambient levels of UV-A, UV-B, and sunlight at a concentration generally used in the treatment of various diseases. Singlet oxygen (¹O₂), superoxide anion radical (O₂.-) generation, DNA damage, and lipid peroxidation are generally observed. The majority of fluoroquinolones produced ¹O₂ and O₂°- under exposure to UV-A, UV-B, and sunlight depending on the concentrations (0 to 60 microg/mL) of the drugs. The formation of the reactive oxygen species (ROS) by the photoexcited drugs may be considered as a possible mechanism of their action.

The phototoprotective potential of Dihydroquercetin was assessed using a method based on Norfloxacin phototoxicity test on fibroblasts NIH-3T3. Dose-dependent photoprotective effect of DHQ was demonstrated because norfloxacin deleterious phototoxic effects decrease and NFX EC50 increases with DHQ increasing concentrations in treatment medium.

### Methods

The study was aimed at evaluating the photoprotective potential of one test item, dihydroquercetin, against the deleterious effects of UVA exposure in presence of phototoxic substances such as Norfloxacin or chlorpromazin.

Briefly, Balb/c 3T3 fibroblasts were maintained in culture for 48 h for formation of monolayers. Norfloxacin 3T3 phototoxicity test was performed in presence or absence of DHQ treatment in different conditions.

After another 24h incubation period, cell viability was determined by Neutral Red uptake. For each condition, cell viability was expressed as percentage relative to untreated solvent controls. Then the dose-response curves (at the EC₅₀ level) obtained for Norfloxacin with or without DHQ treatment was compared to assess DHQ photoprotective potential.

### Results

It has been demonstrated that DHQ increases Norfloxacin EC₅₀ when the treatment is performed at 100µg/mL during UVA exposure. The 2^{nd} experiment shows that DHQ photoprotective-effect is dose-dependent (see table 2).

**Table 2: Dose-dependent photoprotective effect of DHQ**

| **Photoprotective treatment** | **NFX EC 50 (µg/mL)** |
|---|---|
| No DHQ treatment | 55,0 |
| DHQ 25µg/mL | 65,2 |
| DHQ 50µg/mL | 81,9 |
| DHQ 75µg/mL | nd (>100,0) |
| DHQ 100µg/mL | nd (>100,0) |

### Conclusion

Dose-dependent photoprotective effect of DHQ was demonstrated because norfloxacin phototoxic effects decrease and NFX EC₅₀ increases with DHQ increasing concentrations in treatment medium. It should be noticed that similar amounts of para-aminobenzoic acid (PABA) a well known UV filter has no effect.

DHQ clearly protects cells against the photoxicity induced by the photosensitization of norfloxacin upon UVA irradiation. The mechanism by which photosensitization occurs is well known and mainly involves the generation of singlet oxygen and to a lesser extent superoxide anion.

In order to further investigate the mechanism of action of DHQ, the same type of experiments as described above has been performed but using chlorpromazine which is a strong photosensitizer and compared the effect of DHQ with vitamin C which is a potent antioxidant (see protocol in table 3). Chlorpromazine (CPZ) is a neuroleptic drug widely used in medicine because of its tranquilizing and antipsychotic properties. CPZ often causes side effects including cutaneous photosensitization and ocular damage. Chlorpromazine photosensitized strong peroxidation of lipids, which was mainly due to generation of singlet oxygen.

**Table 3: Protocol used to investigate the possible protective effect of dihydroquercetin on chlorpromazine (CPZ)-induced cell photoxicity.**

| **Non-irradiated** | **5J/cm²UVA** | **20J/cm²UVA** |
|---|---|---|
| Dihydroquercetin pretreatment + CPZ (5 doses) | Dihydroquercetin pretreatment + CPZ (5 doses) | Dihydroquercetin pretreatment + CPZ (5 doses) |
| Dihydroquercetin pretreatment 100µg/ml | Dihydroquercetin pretreatment 100µg/ml | Dihydroquercetin pretreatment 100µg/ml |
| CPZ (5 doses) during irradiation | CPZ (5 doses) during irradiation | CPZ (5 doses) during irradiation |
| Not treated (control) | Not treated (control) | Not treated (control) |

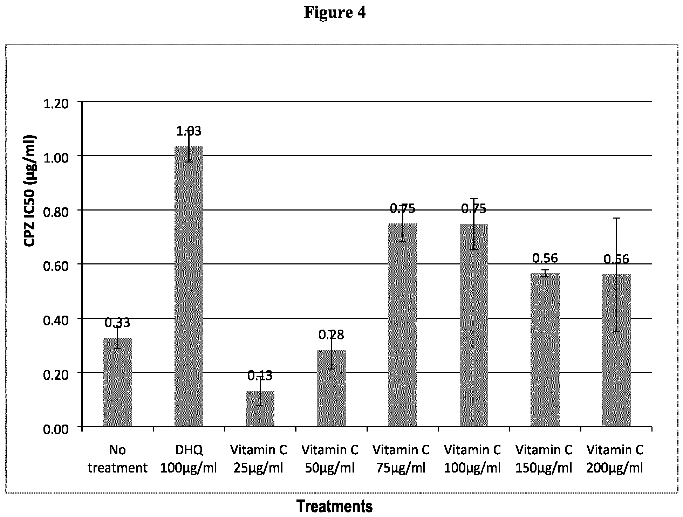

The results in Figure 4 show that DHQ efficiently protects the NIH-3T3 fibroblasts of the phototoxicity induced by chlorpromazine + UVA and that at any concentrations, vitamin C protect the cells to a lesser extent. This suggests that the scavenging effect of superoxide anion *per se* is not the major mechanism of photoprotection and that the quenching of singlet oxygen remains the single plausible hypothesis.

It is of interest to note that UVA light (320-400 nm) has been shown to produce deleterious biological effects in tissue due to the generation of singlet oxygen by substances like flavin derivatives or urocanic acid.

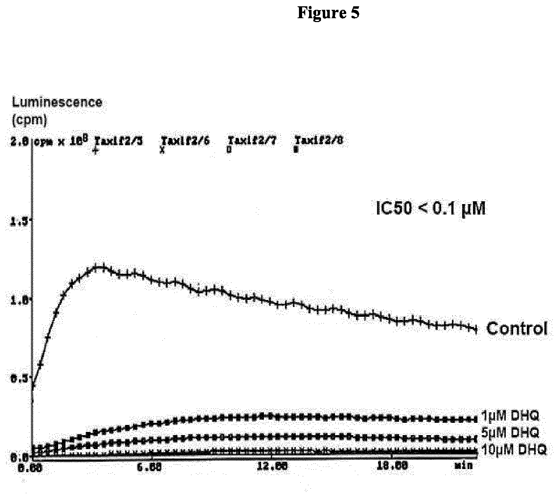

### Dihydroquercetin mediated inhibition of polymorphonuclear neutrophils oxidative burst (Figure 5)

Inhibitory effect of DHQ on the reactive oxygen species (ROS) production resulting from the neutrophils activation by 100 ng/ml PMA. ROS production is measured using a chemiluminescence standard procedure: briefly, one hundred microliters of human neutrophils (4 × 106/mL) were primed with TNF-α at 37°C for 25 minutes. 100 µL luminol (1 µM final concentration) and HRP (62.5 U/mL final concentration) in HBSS were added, and 150-µL aliquots were transferred to a prewarmed 96-well luminometer plate. Light emission was recorded by a Berthold MicroLumat Plus luminometer (Berthold Technologies, Hartfordshire, United Kingdom) (data output is in relative light units per second)

### Bisabolol mediated inhibition of polymorphonuclear neutrophils myeloperoxidase release (Table 4)

Neutrophils were stimulated with formyl-methionyl-leucyl-phenylalanine for 45 minutes at 37° C. MPO released from neutrophils were assayed by using an MPO enzyme immunoassay.

**Table 4: Bisabolol-mediated Inhibition of neutrophils degranulation assessed by the release of myeloperoxidase (MPO). human neutrophils (4 × 106/mL).**

| Bisabolol Concentrations (µM) | MPO release (U/mL) |
|---|---|
| 0.00 | 800 |
| 20 | 810 |
| 40 | 500 |
| 100 | 250 |
| 200 | 248 |

### References

Baier Jürgen, Tim Maisch, Max Maier, Eva Engel, Michael Landthaler and Wolfgang Bäumler, Singlet Oxygen Generation by UVA Light Exposure of Endogenous Photosensitizers Biophys J. Aug 15, 2006; 91(4): 1452-1459.
Chan Paul, Juei-Tang Cheng, Jen-Chen Tsa, Gi-Shih Lien, Fu-Chean Chen, Pai-Feng Kao, Ju-Chi Liu, Yi-Jen Chen, Min-Hsing Hsieh, Effect of catechin on the activity and gene expression of superoxide dismutase in cultured rat brain astrocytes Neuroscience Letters Volume 328, Issue 3, 2002, 281-284.
Choe E. and D.B. Min Kinetics for Singlet Oxygen Formation by Riboflavin Photosensitization and the Reaction between Riboflavin and Singlet Oxygen R. Huang, Version of Record online: 31 MAY 2006 The Journal of Food Science.
Dalle Carbonare M, Pathak MA. Skin photosensitizing agents and the role of reactive oxygen species in photoaging. J Photochem Photobiol B. 1992 Jun 30;14(1-2):105-24.
Drucker AM, Rosen CF. Drug-induced photosensitivity: culprit drugs, management and prevention. Drug Saf. 2011 Oct 1;34(10):821-37.
Nishikawa H, Wakano K, Kitani S. Inhibition of NADPH oxidase subunits translocation by tea catechin EGCG in mast cell. Biochem Biophys Res Commun. 2007 Oct 19;362(2):504-9.
Rocha NF, Rios ER, Carvalho AM, Cerqueira GS, Lopes Ade A, Leal LK, Dias ML, de Sousa DP, de Sousa FC. Anti-nociceptive and anti-inflammatory activities of (-)-α-bisabolol in rodents. Naunyn Schmiedebergs Arch Pharmacol. 2011 Dec; 384(6): 525-33.
Selvaag E., Helle Anholt, Johan Moan, Per Thune Inhibiting effects of antioxidants on drug-induced phototoxicity in cell cultures Investigations with sulphonamide-derived oral antidiabetics and diuretics Journal of Photochemistry and Photobiology B: Biology, Volume 38, Issue 1, March 1997, Pages 88-93

## Claims

1. A pharmaceutical composition comprising therapeutically effective amounts of
- dihydroflavone or dihydroflavonol,
- (+)-catechin or a derivative thereof,
- terpene alcohol, and
- pharmaceutically acceptable excipients and/or carriers.

2. The pharmaceutical composition of claim 1, wherein dihydroflavone or dihydroflavonol is selected from the group comprising Dihydroquercetin (DHQ), Dihydrokaempferol, Butin, Eriodictyol, Hesperetin, Hesperidin, Homoeriodictyol, Isosakuranetin, Naringenin, Naringin, Pinocembrin, Poncirin, Sakuranetin, Sakuranin, Sterubin.

3. The pharmaceutical composition of any one of claims 1-2, comprising therapeutically effective amount of (+)-catechin.

4. The pharmaceutical composition of any one of claims 1-3, wherein terpene alcohol is selected from the group comprising cedrenol, cedrols, geraniol, nerolidol, bisabolols, citronellol, nerol, terpineol, linalool, menthol, pulegol, carveol, pinocampheol, myrcenol, isopulegol, farnesol, lanceol, santalols, vetiverol, viridiflorol, valerianol, tumerols, patchoulol, occidol, nootkatol, jinkoh eremol, hanamyol, guaicol germacradienol, fokienol, eudesmols, and cadinols, an active optical or steric isomer of these compounds and combinations of one or more thereof.

5. The pharmaceutical composition according to any one of claims 1-4, comprising
- Dihydroquercetin,
- (+)-catechin,
- Bisabolol, and
- pharmaceutically acceptable excipients and/or carriers.

6. The pharmaceutical composition of any one of claims 1-5 for use in a method for treating or lessening the symptoms of, or preventing the symptoms of drug-induced phototoxicity or photosensitivity.

7. The pharmaceutical composition for use of clam 6, wherein the symptoms of drug-induced phototoxicity or photosensitivity are selected from the group comprising erythema, pain, prickling, rash, burning sensation of areas of skin exposed to the sunlight, and/or the appearance of a sunburn-like reaction on areas of skin exposed to the sunlight.

8. The pharmaceutical composition for use of any one of claims 6-7, wherein the method comprises applying the pharmaceutical composition to the affected skin of a patient once a day.

9. The pharmaceutical composition for use of any one of claims 6-7, wherein the method comprises applying the pharmaceutical composition to the affected skin of a patient multiple times a day.
